# EUROPEAN PATENT APPLICATION

(11) **EP 4 729 601 A1**
(43) Date of publication of application: **22.04.2026**
(21) Application number: 24823135.9
(22) Date of filing: 09.05.2024
(51) Int. Cl.: C12N 1/00, C12M 1/00

(54) **CELL CULTURE METHOD**

(30) Priority: 15.06.2023 JP 2023098483
(71) Applicant: SHIMADZU CORPORATION, Kyoto-shi, Kyoto 604-8511 (JP)
(72) Inventor: HASHIMOTO, Toyoyuki, Kyoto-shi, Kyoto 604-8511 (JP); TAKAHASHI, Mikako, Kyoto-shi, Kyoto 604-8511 (JP); SONOMURA, Kazuhiro, Kyoto-shi, Kyoto 604-8511 (JP)
(74) Representative: Müller-Boré & Partner Patentanwälte PartG mbB
(86) International application number: PCT/JP2024/017243
(87) International publication number: WO 2024/257513

(57) **Abstract**

A co-culture vessel (100) includes a container body (110) and a cell culture insert (120) capable of storing a medium therein. The cell culture insert (120) includes an oxygen-permeable membrane (122), the membrane (122) separating the container body (110) and the cell culture insert (120). A cell culture method includes: (a) a step of culturing cells so as to cover the membrane (122) inside the cell culture insert (120); (b) a step of introducing a first medium (161) exhibiting a first color into the container body (110); (c) a step of introducing a second medium (162) containing bacteria and exhibiting a second color different from the first color into the cell culture insert (120); and (d) a step of co-culturing the cells and the bacteria in a state where the membrane (122) is arranged so as to be in contact with the first medium.

## Description

### TECHNICAL FIELD

The present disclosure relates to a cell culture method.

### BACKGROUND ART

Cell culture systems that simulate the intestinal environment of living organisms are being developed. International Publication No. WO 2022/009672 (Patent Literature 1) discloses a system for co-culturing intestinal epithelial cells and bacteria contained in a medium within an anaerobic medium, using a co-culture vessel in which an anaerobic medium and an aerobic medium are separated by a porous membrane (membrane).

### CITATION LIST

### PATENT LITERATURE

[Patent Literature 1] International Publication No. WO 2022/009672

### SUMMARY OF INVENTION

### TECHNICAL PROBLEM

In the cell culture systems described above, after the culture process is completed, each medium is transferred to a separate container such as an Eppendorf tube, and analysis of metabolites and the like is performed in a subsequent processing step such as analysis. At this time, if the transferred media are mixed up, it will lead to erroneous analysis results, making accurate analysis and evaluation impossible.

The present disclosure has been made to solve such a problem, and an object thereof is to prevent mix-ups of respective media after culturing in a cell culture method for co-culturing cells and bacteria using two different media.

### SOLUTION TO PROBLEM

A method according to the present disclosure relates to a method for co-culturing a first biological element and a second biological element using a co-culture vessel. The co-culture vessel includes a first chamber and a second chamber capable of storing a medium therein. The second chamber includes an oxygen-permeable membrane, the membrane separating the first chamber and the second chamber. The method includes: (a) a step of arranging the first biological element so as to cover the membrane inside the second chamber; (b) a step of introducing a first medium exhibiting a first color into the first chamber; (c) a step of introducing a second medium containing the second biological element and exhibiting a second color different from the first color into the second chamber; and (d) a step of culturing the first biological element and the second biological element in a state where the membrane is arranged so as to be in contact with the first medium.

### ADVANTAGEOUS EFFECTS OF INVENTION

In the cell culture method according to the present disclosure, media exhibiting different colors from each other are used as the two media for co-culture. This makes it possible to easily distinguish which medium is which when each medium is taken out after the completion of culturing. Therefore, in a cell culture method for co-culturing different biological elements using two different media, it is possible to prevent mix-ups of the respective media after culturing.

### BRIEF DESCRIPTION OF DRAWINGS

[FIG. 1] FIG. 1 is a configuration diagram of a cell culture system used in the cell culture method of Embodiment 1.
[FIG. 2] FIG. 2 is a flowchart showing a culture process in the cell culture method.
[FIG. 3] FIG. 3 is a diagram for explaining a state where the media are collected after the completion of culturing.
[FIG. 4] FIG. 4 is a diagram showing an example of the state of the media when cells are damaged during culturing.
[FIG. 5] FIG. 5 is a flowchart for explaining an example of a sample processing step after the completion of culturing.
[FIG. 6] FIG. 6 is a configuration diagram of a cell culture system used in the cell culture method of Embodiment 2.
[FIG. 7] FIG. 7 is an exploded perspective view of a co-culture device used in the cell culture system of FIG. 6.

### DESCRIPTION OF EMBODIMENTS

Hereinafter, embodiments of the present disclosure will be described in detail with reference to the drawings. In the drawings, the same or corresponding parts are denoted by the same reference numerals, and a description thereof will not be repeated.

In this specification, biological cells and microorganisms are collectively referred to as biological elements. An example of biological cells is organ-constituting cells (a specific example is organ-constituting cells such as organoids). An example of microorganisms is fungi or bacteria.

### [Embodiment 1]

### (Configuration of Cell Culture System)

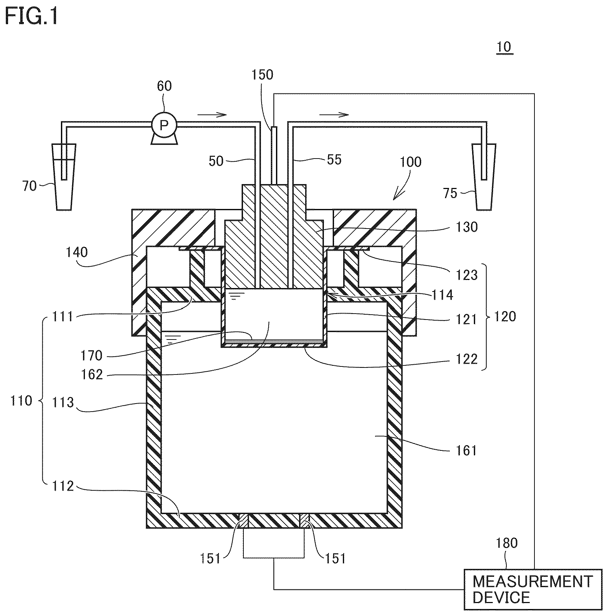

FIG. 1 is a configuration diagram of a cell culture system 10 used in the cell culture method of Embodiment 1. The cell culture system 10 includes a cell culture apparatus 100, a pump 60, medium containers 70 and 75, and a measurement device 180. The cell culture system 10 is, for example, placed in an anaerobic chamber, and the surroundings are maintained in an anaerobic environment.

The cell culture apparatus 100 includes a container body 110, a cell culture insert 120, lid members 130 and 140, and an electrode 150. The container body 110 is a container having a substantially cylindrical shape and has a top wall 111, a bottom wall 112, and a side wall 113. The side wall 113 connects the top wall 111 and the bottom wall 112. The container body 110 is formed of, for example, a resin material. A medium 161 (first medium) is stored inside the container body 110. By attaching the cell culture insert 120, in which cells (organ-constituting cells) 170 are cultured in a sheet form, to the container body 110 in an atmospheric environment, the inside of the container body 110 is maintained in an atmospheric environment (aerobic environment). The "container body 110" of the embodiment corresponds to the "first chamber" in the present disclosure, and the "cell culture insert 120" corresponds to the "second chamber" in the present disclosure.

An opening 114 that penetrates along the thickness direction of the top wall 111 is formed in the top wall 111 of the container body 110. As will be described later, the cell culture insert 120 is placed in this opening 114. Although FIG. 1 shows a configuration example in which the top wall 111 is formed integrally with the side wall 113, the top wall 111 may be formed as a separate member from the side wall 113.

An electrode 151 is embedded in the bottom wall 112 of the container body 110. The electrode 151 is exposed on the inner and outer surfaces of the bottom wall 112. Inside the container body 110, the electrode 151 is in contact with the medium 161, allowing the electrode 151 and the medium 161 to be electrically connected.

The cell culture insert 120 is a container having a substantially cylindrical shape and has a tubular portion 121 forming the side wall of the container, a membrane 122 forming the bottom wall of the container, and a flange portion 123. The lower end of the tubular portion 121 is closed by the membrane 122. The membrane 122 is formed of an oxygen-permeable member such as, for example, a polycarbonate track-etched membrane. The membrane 122 may be a porous membrane made of another material such as PET (polyethylene terephthalate) or a collagen vitrigel membrane.

The upper end of the tubular portion 121 is open and sealed by a removable lid member 130. A flange portion 123 is provided at the upper end of the tubular portion 121, projecting in the outer circumferential direction.

The cell culture insert 120 is inserted into the opening 114 of the top wall 111 in the container body 110. At this time, the flange portion 123 of the cell culture insert 120 is supported by the top wall 111. Then, the cell culture insert 120 is fixed to the container body 110 by a detachable lid member 140. In a state where the cell culture insert 120 is fixed, the membrane 122 on the lower surface of the cell culture insert 120 is immersed in the medium 161.

A medium 162 is stored inside the cell culture insert 120. The dissolved oxygen concentration of the medium 162 is lower than the dissolved oxygen concentration of the medium 161 in the container body 110. That is, the medium 161 is an aerobic medium, and the medium 162 is an anaerobic medium. The medium 162 contains a biological element. In the example of Embodiment 1, the biological element contained in the medium 162 is an anaerobic bacteria. The anaerobic bacteria may be a facultative anaerobe such as a lactic acid bacterium, or an obligate anaerobe such as a bifidobacterium. The anaerobic bacteria corresponds to the "second biological element" in the present disclosure.

Pipes 50 and 55 pass through the lid member 130. By a pump 60 disposed in the pipe 50, the medium 162 (second medium) stored in a medium container 70 outside the apparatus is introduced into the cell culture insert 120. The pipe 55 is a pipe for discharging the medium 162 in the cell culture insert 120. The pipe 55 leads to a medium container 75 for storing waste medium. The medium 162 that overflows from the cell culture insert 120 is discharged to the medium container 75 through the pipe 55.

A biological element is cultured on the inner surface of the membrane 122. In the example of Embodiment 1, the biological element is cells (organ-constituting cells) 170. The cells 170 are, for example, intestinal epithelial cells that form a tight junction on the membrane 122. A specific example of the cells 170 is Caco-2 cells. The membrane 122 has oxygen permeability as described above. Oxygen in the medium 161 is supplied to the cells 170 through the membrane 122. By co-culturing cells and bacteria in the anaerobic medium 162 in this manner, the in-vivo intestinal environment can be simulated. The "cells 170" correspond to the "first biological element" in the present disclosure.

An electrode 150 is further disposed on the lid member 130. Although not shown in FIG. 1, one end of the electrode 150 is in contact with the medium 162 stored in the cell culture insert 120 and is electrically connected to the medium 162.

The electrode 150 disposed on the lid member 130 of the cell culture insert 120 and the electrode 151 embedded in the bottom wall 112 of the container body 110 are connected to a measurement device 180 provided outside the apparatus. The measurement device 180 measures the electrical resistance value between the electrode 150 and the electrode 151 by applying a voltage between the electrode 150 and the electrode 151. The measurement of the electrical resistance value in the measurement device 180 is performed, for example, by a four-terminal method.

The resistance value between the electrodes changes depending on the presence or absence of the formation of tight junctions of the cells 170 cultured on the membrane 122. Therefore, by monitoring the electrical resistance value during culturing, it is possible to determine whether or not the tight junctions of the cells 170 are normally formed.

Although not shown in FIG. 1, an oxygen sensor for detecting the dissolved oxygen concentration of the medium 161 and the medium 162 may be provided. By detecting the dissolved oxygen concentration of each medium with the oxygen sensor, the growth status of bacteria during co-culture can be monitored.

When the co-culture process of cells and bacteria using the cell culture system as described above is completed, each medium is collected in another container such as an Eppendorf tube and stored for a predetermined period in a freezer, or a subsequent analysis process is performed. At this time, if the appearance (color) of the collected media is similar, there is a possibility that the user may mix up the aerobic medium and the anaerobic medium during handling of the containers.

In addition, during cell culture, a part of the cells and the membrane may be damaged, and one medium may enter the other medium, resulting in mixing of the aerobic medium and the anaerobic medium. That is, in such a case, the culture process itself is in a failed state. However, if the colors of the media during culturing are similar, it is not possible to detect the state where the media are mixed, and the media in the failed culture state may be analyzed.

If an analysis process is performed while a mix-up of the media after collection and/or mixing of the media during culturing has occurred, the results will be erroneous, and there is a possibility of misjudging the experimental results and evaluation.

Therefore, in the present Embodiment 1, the colors of the aerobic side medium 161 and the anaerobic side medium 162 are made to be different from each other so that they can be visually distinguished by the user. This makes it possible to easily determine which is the aerobic medium and which is the anaerobic medium after the media are collected in containers such as Eppendorf tubes. Furthermore, by making the colors of the respective media different during culturing, it is possible to easily detect that the media have mixed during culturing.

It should be noted that it is sufficient that at least one of the two media is colored, and either one may be colorless. The coloring of the medium can be performed, for example, using a coloring agent that has little effect on the culture process.

The color of the medium may be a fixed color that does not change before and after culturing, or it may be one that changes color depending on the state of the medium. For example, when an indicator that changes color depending on pH, such as methyl orange, phenolphthalein (PP), and bromothymol blue (BTB), is added to the medium, the color of the medium may change due to the change in the pH of the medium accompanying the progress of culturing.

In order to prevent mix-ups of the collected media, it is not essential that the colors of the two media be different from the beginning of the culture process; it is sufficient that the colors of the two media are different at the time of completion of culturing due to the passage of time or a change in the state of the media. On the other hand, in order to detect the mixed state of the media during culturing, it is preferable that the colors of the media be different from the initial stage of culturing.

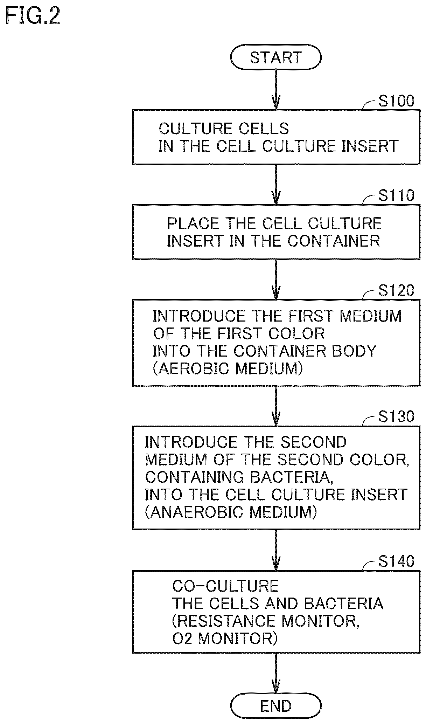

FIG. 2 is a flowchart showing an example of a culture process performed using the cell culture system 10 of FIG. 1. Each step of the flowchart of FIG. 2 may be performed by a user's manual operation, or some or all of the steps may be automatically performed using a handling device (not shown) or the like.

Referring to FIG. 2, in step (hereinafter, step is abbreviated as S) 100, cells are cultured over the entire surface of the membrane 122 in the cell culture insert 120. Thereafter, in S110, the cell culture insert 120 is placed in the opening 114 of the container body 110.

In S120, the medium 161 of the first color is introduced into the container body 110. At this time, the medium 161 is introduced to a height at which the membrane 122 on the lower surface of the cell culture insert 120 is immersed in the medium 161. Then, in S130, the medium 162 of the second color containing bacteria is introduced into the cell culture insert 120. As described with reference to FIG. 1, new medium 162 is supplied from the medium container 70 into the cell culture insert 120 by the pump 60 even during culturing.

In S120 and S130, the media 161 and 162 to be used may be those that are already colored in a commercially available state. Alternatively, a step of coloring a colorless medium using a coloring agent and/or an indicator may be included prior to S120 and S130.

When the media are introduced into the container body 110 and the cell culture insert 120, in S140, the cells and bacteria are cultured for a predetermined time while monitoring the electrical resistance and the dissolved oxygen concentration with the measurement device 180.

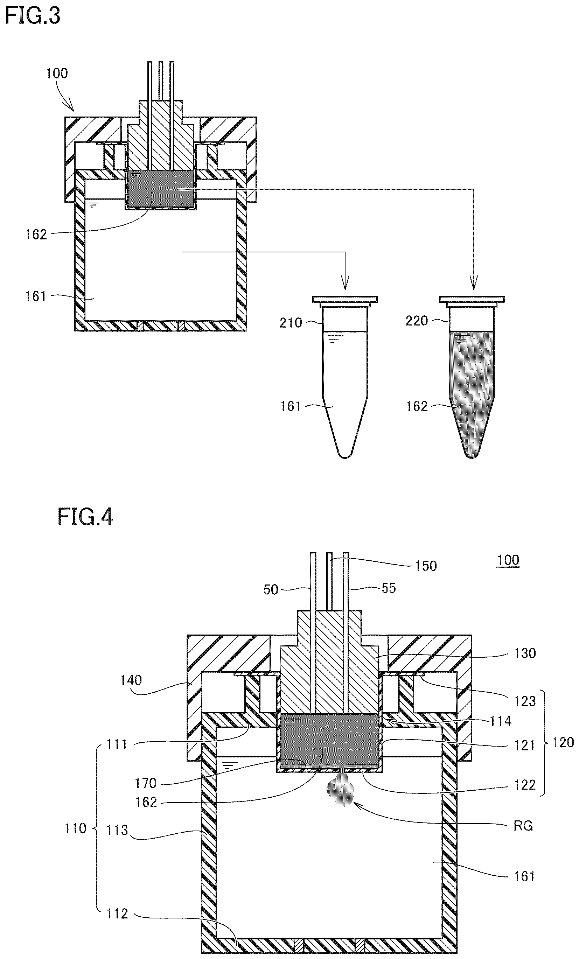

FIG. 3 is a diagram for explaining a state where the media 161 and 162 are collected after the completion of culturing. FIG. 3 shows a state where the color of the medium 161 and the color of the medium 162 are different at the stage where culturing is completed. In the example of FIG. 3, the color of the medium 161 is shown lightly, and the color of the medium 162 is shown darkly. Even when the media 161 and 162 are respectively collected in Eppendorf tubes 210 and 220, it is possible to determine whether it is the medium 161 or the medium 162 by the difference in the color of each medium.

FIG. 4 is a diagram showing an example of the state of the media when the cells 170 and the membrane 122 are damaged during culturing. For example, if the pressure of the medium 162 is higher than the pressure of the medium 161, when the cells 170 and the membrane 122 are damaged during culturing, a part of the medium 162 in the cell culture insert 120 leaks into the medium 161 from the damaged portion, as in a region RG of FIG. 4. At this time, if the color of the medium 161 and the color of the medium 162 are different, the medium 162 that has leaked into the medium 161 can be easily detected. If the pressure of the medium 161 is higher than the pressure of the medium 162, when the cells 170 and the membrane 122 are damaged during culturing, the medium 161 leaks into the medium 162.

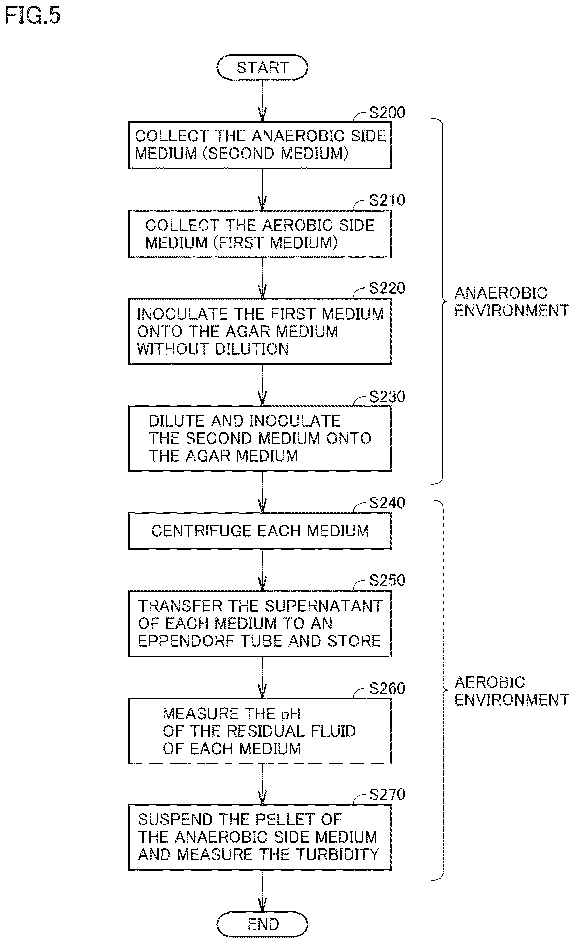

FIG. 5 is a flowchart for explaining an example of a sample processing step after the completion of culturing. The processing of S200 to S240 in the flowchart of FIG. 5 is executed in an anaerobic environment, and the processing of S250 to S280 is executed in an aerobic environment.

Referring to FIG. 5, after the completion of culturing, in S200, a specific amount of the medium 162, which is an anaerobic medium, is collected in a container in an anaerobic environment. In S210, a specific amount of the medium 161, which is an aerobic medium, is collected in another container. If necessary, each medium collected in the anaerobic environment is allowed to stand for a predetermined time.

Thereafter, for the medium 161, in order to confirm that no bacteria are present in the aerobic environment, it is inoculated onto an agar medium without dilution (S220). On the other hand, for the medium 162, since it is a medium co-cultured with bacteria and it is necessary to count the bacteria, it is inoculated onto an agar medium after being diluted (S230). The dilution ratio varies depending on conditions such as the type of bacteria and/or the turbidity of the medium.

When the inoculation onto the agar medium is completed, the containers containing the remaining media are taken out to an aerobic environment, and each medium is centrifuged using a centrifuge in S240. In S250, the supernatant of each medium after centrifugation is transferred to another container such as an Eppendorf tube and stored in a predetermined environment.

Thereafter, in S260, the residual liquid of each medium is collected, and the pH of each medium is measured. In S270, the precipitated pellet of the medium 162 is suspended in an appropriate amount of distilled water, and the turbidity is measured.

When performing the sample processing steps as shown in FIG. 5, if the two media are mixed up or if the media are mixed during culturing, not only is the work up to that point wasted, but also the desired analysis results cannot be obtained. As in the present Embodiment 1, by making the two media have different colors that can be visually distinguished by the user, the media after collection can be easily identified, so that mix-ups of the two media can be prevented. Furthermore, even when the medium leaks due to damage to the cells or the like during culturing, the leakage state of the medium can be easily detected, and experimental failure can be discovered at an early stage.

### [Embodiment 2]

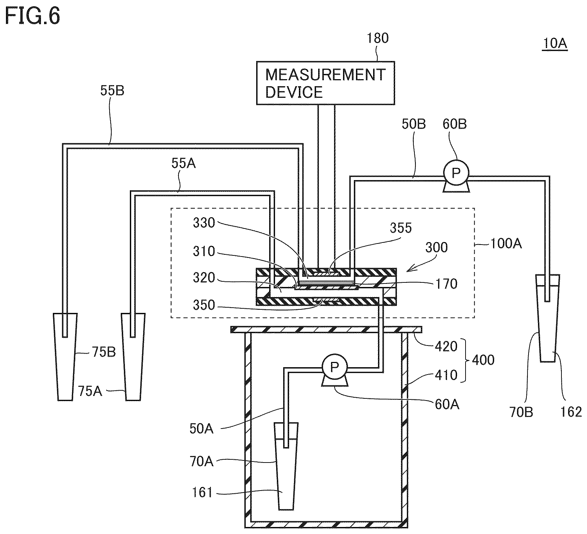

In Embodiment 2, another configuration example of the cell culture apparatus will be described. FIG. 6 is a configuration diagram of a cell culture system 10A used in the cell culture method of Embodiment 2.

Referring to FIG. 6, the cell culture system 10A includes a cell culture apparatus 100A, pumps 60A and 60B, medium containers 70A, 70B, 75A, and 75B, a measurement device 180, and a sealed container 400. The cell culture system 10A is, for example, placed in an anaerobic chamber, and the surroundings are maintained in an anaerobic environment.

The cell culture apparatus 100A includes a co-culture device 300. In the co-culture device 300, two channels 320 and 330 adjacent via a membrane 310 are formed. The channel 320 is a path through which an aerobic medium (medium 161) flows, and the channel 330 is a path through which an anaerobic medium (medium 162) flows.

Cells 170 are cultured on the surface of the membrane 310 on the channel 320 side. Oxygen is supplied to the cells 170 from the medium 161 flowing through the channel 320 via the membrane 310.

The sealed container 400 is a container whose internal space can be sealed. The sealed container 400 includes a body portion 410 and a lid member 420. The body portion 410 has a cylindrical shape with one end closed by a bottom wall and the other end open. A detachable lid member 420 is attached to the open end of the body portion 410. The internal space of the sealed container 400 is maintained in an atmospheric environment, that is, an aerobic environment.

Inside the sealed container 400, a medium container 70A in which the medium 161 is stored, a pipe 50A, and a pump 60A provided in the pipe 50A are disposed. The pipe 50A is connected from the medium container 70A to the channel 320 of the co-culture device 300. By driving the pump 60A, the medium 161 in the medium container 70A is supplied to the channel 320 through the pipe 50A. The medium 161 that has passed through the channel 320 is discharged to a waste liquid medium container 75A through a pipe 55A.

The medium 162 is stored in a medium container 70B disposed in an anaerobic environment. The medium container 70B is connected to the channel 330 of the co-culture device 300 by a pipe 50B. By driving a pump 60B provided in the pipe 50B, the medium 162 in the medium container 70B is supplied to the channel 330 through the pipe 50B. The medium 162 that has passed through the channel 330 is discharged to a waste liquid medium container 75B through a pipe 55B.

In the co-culture device 300, an electrode 350 is disposed so as to be exposed in the channel 320, and an electrode 355 is disposed so as to be exposed in the channel 330. The electrode 350 can be electrically connected to the medium 161 flowing in the channel 320, and the electrode 355 can be electrically connected to the medium 162 flowing in the channel 330. The electrodes 350 and 355 are connected to the measurement device 180. By applying a voltage to the electrodes 350 and 355 from the measurement device 180, the electrical resistance value between the electrode 350 and the electrode 355 can be monitored.

As in the case of Embodiment 1, an oxygen sensor for detecting the dissolved oxygen concentration of the media 161 and 162 flowing through each channel may be provided.

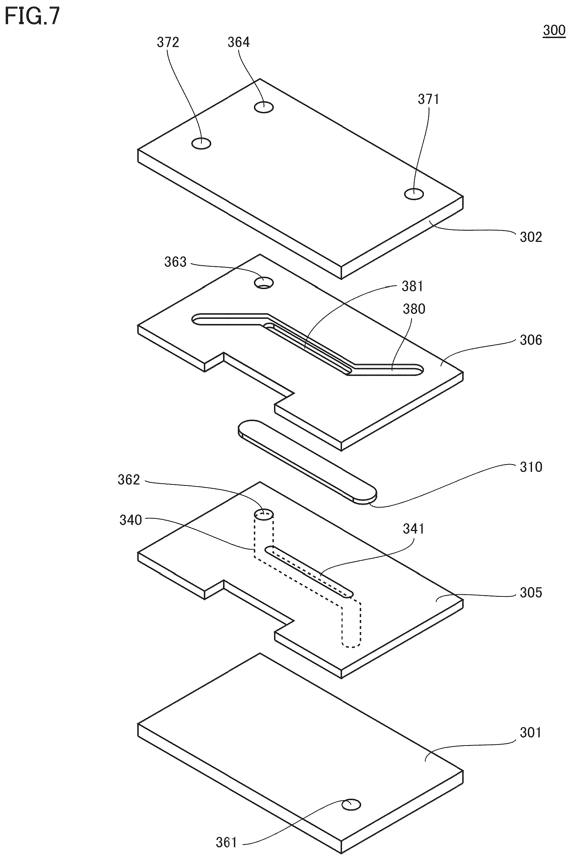

Next, a detailed configuration of the co-culture device 300 in FIG. 6 will be described. FIG. 7 is an exploded perspective view of the co-culture device 300 used in the cell culture system 10A of FIG. 6.

Referring to FIG. 7, the co-culture device 300 includes plate-shaped glass plates 301 and 302, and plate-shaped resin sheets 305 and 306 disposed between the glass plates 301 and 302. The glass plates 301 and 302 and the resin sheets 305 and 306 are stacked in the order of the glass plate 301, the resin sheet 305, the resin sheet 306, and the glass plate 302.

A specific example of the resin sheets 305 and 306 is, for example, silicone rubber. The bonding between the glass plates and the resin sheets and the bonding between the resin sheets are performed, for example, by pressurizing the bonding surfaces in a state where the bonding surfaces are activated by oxygen plasma.

A through hole 361 penetrating in the thickness direction is formed in the glass plate 301. The pipe 50A is connected to the through hole 361. That is, the through hole 361 serves as an inlet of the channel 320 through which the medium 161 flows.

A groove 340 is formed on the surface of the resin sheet 305 facing the glass plate 301. This groove 340 and the surface of the glass plate 301 form the channel 320 in FIG. 6. One end of the groove 340 communicates with the through hole 361 of the glass plate 301. A through hole 362 penetrating in the thickness direction of the resin sheet 305 is formed at the other end of the groove 340.

In the resin sheet 306 and the glass plate 302, through holes 363 and 364 are formed at positions corresponding to the through hole 362 of the resin sheet 305, respectively. The pipe 55A is connected to the through hole 364 of the glass plate 302. That is, the through hole 364 of the glass plate 302 serves as an outlet of the channel 320 through which the medium 161 flows.

A groove 380 is formed on the surface of the resin sheet 306 facing the glass plate 302. This groove 380 and the surface of the glass plate 302 form the channel 330 in FIG. 6.

In the glass plate 302, a through hole 371 penetrating in the thickness direction is formed at a position corresponding to one end of the groove 380, and a through hole 372 penetrating in the thickness direction is formed at a position corresponding to the other end of the groove 380. The pipe 50B is connected to the through hole 371 of the glass plate 302. The pipe 55B is connected to the through hole 372 of the glass plate 302. That is, the through holes 371 and 372 of the glass plate 302 serve as an inlet and an outlet of the channel 330 through which the medium 162 flows, respectively.

When viewed in a plan view from the normal direction of the resin sheet 305, a part of the groove 340 formed in the resin sheet 305 overlaps with the groove 380 formed in the resin sheet 306 in parallel. A slit 341 penetrating in the thickness direction is formed in the overlapping portion of the resin sheet 305. In the groove 380 of the resin sheet 306, a slit 381 is formed at a position facing the slit 341. The membrane 310 is disposed between the slit 341 and the slit 381.

With such a configuration, a configuration is realized in which two channels 320 and 330 adjacent via the membrane 310 are connected. That is, in the cell culture system 10A used in the cell culture method of Embodiment 2, the channel 320 in FIG. 6 corresponds to the container body 110 of the cell culture system 10 of Embodiment 1, and the channel 330 corresponds to the cell culture insert 120 of the cell culture system 10. That is, the "channel 320" and the "channel 330" in Embodiment 2 correspond to the "first chamber" and the "second chamber" in the present disclosure, respectively.

Also when this cell culture system 10A is used, by making the color of the aerobic medium different from the color of the anaerobic medium, it is possible to prevent mix-ups of the two media in the processing after the completion of culturing. In addition, mixing of the media during culturing can be easily detected.

### [Supplementary Note]

It is understood by those skilled in the art that the plurality of exemplary embodiments described above are specific examples of the following aspects.

(Item 1) A cell culture method according to one aspect relates to a method for co-culturing a first biological element and a second biological element using a co-culture vessel. The co-culture vessel includes a first chamber and a second chamber capable of storing a medium therein. The second chamber includes an oxygen-permeable membrane, the membrane separating the first chamber and the second chamber. The cell culture method includes: (a) a step of culturing the first biological element so as to cover the membrane inside the second chamber; (b) a step of introducing a first medium exhibiting a first color into the first chamber; (c) a step of introducing a second medium containing the second biological element and exhibiting a second color different from the first color into the second chamber; and (d) a step of co-culturing the first biological element and the second biological element in a state where the membrane is arranged so as to be in contact with the first medium.

According to the cell culture method of Item 1, in a co-culture vessel, by performing co-culture using two media (an aerobic medium and an anaerobic medium) exhibiting different colors from each other, it is possible to prevent mix-ups of the two media when each medium is collected in a separate container after the completion of co-culturing. In addition, when the media are mixed during culturing due to damage to cells or the like, the abnormal state can be easily discovered.

(Item 2) In the cell culture method according to Item 1, the second color is a color that can be visually distinguished from the first color by a user.

According to the cell culture method of Item 2, by making the color of each medium a color that can be visually distinguished by the user, the two media can be easily distinguished.

(Item 3) The cell culture method according to Item 1 or 2 further includes a step of coloring the first medium to the first color, and a step of coloring the second medium to the second color.

According to the method of Item 3, when the original media are colorless, by coloring the media with a coloring agent or the like, the two media can be easily distinguished.

(Item 4) In the cell culture method according to Item 1 or 2, at least one of the first medium and the second medium includes an indicator whose color changes depending on the state of the medium.

According to the cell culture method of Item 4, when the state of the medium (for example, pH) changes during the culture process, by using an indicator whose color changes depending on the state of the medium, the change in the state of the medium can be confirmed, and the media after culturing can be easily distinguished by the difference in color.

(Item 5) In the cell culture method according to any one of Items 1 to 4, the dissolved oxygen concentration of the first medium is higher than the dissolved oxygen concentration of the second medium.

According to the cell culture method of Item 5, the first medium can be used as an aerobic medium, and the second medium can be used as an anaerobic medium.

(Item 6) In the cell culture method according to any one of Items 1 to 5, the second chamber is a cell culture insert further including a tubular portion, wherein the membrane is disposed so as to close a lower end of the tubular portion.

According to the cell culture method of Item 5, a widely used cell culture insert can be used as the second chamber.

(Item 7) In the cell culture method according to any one of Items 1 to 6, the first biological element is an organ-constituting cell, and the second biological element is a microorganism.

According to the cell culture method of Item 7, the cell culture method of the present disclosure can be applied to co-culture of organ-constituting cells and microorganisms.

The embodiments disclosed this time should be considered to be illustrative in all respects and not restrictive. The scope of the present invention is indicated by the claims rather than by the description of the embodiments above, and all changes that come within the meaning and range of equivalency of the claims are intended to be embraced therein.

### REFERENCE SIGNS LIST

10, 10A: Cell culture system
50, 50A, 50B, 55, 55A, 55B: Pipe
60, 60A, 60B: Pump
70, 70A, 70B, 75, 75A, 75B: Medium container
100, 100A: Cell culture apparatus
110: Container body
111: Top wall
112: Bottom wall
113: Side wall
114: Opening
120: Cell culture insert
121: Tubular portion
122, 310: Membrane
123: Flange portion
130, 140, 420: Lid member
150, 151, 350, 355: Electrode
161, 162: Medium
170: Cell
180: Measurement device
210, 220: Eppendorf tube
300: Co-culture device
301, 302: Glass plate
305, 306: Resin sheet
320, 330: Channel
340, 380: Groove
341, 381: Slit
361-364, 371, 372: Through hole
400: Sealed container
410: Body portion
RG: Region

## Claims

1. A cell culture method for co-culturing a first biological element and a second biological element using a co-culture vessel, wherein
the co-culture vessel includes a first chamber and a second chamber capable of storing a medium therein,
the second chamber includes an oxygen-permeable membrane, the membrane separating the first chamber and the second chamber, and
the cell culture method comprises:
a step of culturing the first biological element so as to cover the membrane inside the second chamber;
a step of introducing a first medium exhibiting a first color into the first chamber;
a step of introducing a second medium containing the second biological element and exhibiting a second color different from the first color into the second chamber; and
a step of co-culturing the first biological element and the second biological element in a state where the membrane is arranged so as to be in contact with the first medium.

2. The cell culture method according to claim 1, wherein the second color is a color that can be visually distinguished from the first color by a user.

3. The cell culture method according to claim 1, further comprising:
a step of coloring the first medium to the first color; and
a step of coloring the second medium to the second color.

4. The cell culture method according to claim 1, wherein at least one of the first medium and the second medium includes an indicator whose color changes depending on a state of the medium.

5. The cell culture method according to claim 1, wherein a dissolved oxygen concentration of the first medium is higher than a dissolved oxygen concentration of the second medium.

6. The cell culture method according to claim 1, wherein the second chamber is a cell culture insert further including a tubular portion, and the membrane is disposed so as to close a lower end of the tubular portion.

7. The cell culture method according to claim 1, wherein
the first biological element is an organ-constituting cell, and
the second biological element is a microorganism.
